# EUROPÄISCHE PATENTSCHRIFT

(11) **EP 0 970 104 B1**
(45) Veröffentlichungstag und Bekanntmachung des Hinweises auf die Patenterteilung: **22.11.2006**
(21) Anmeldenummer: 98924026.2
(22) Anmeldetag: 18.03.1998
(51) Int. Cl.: C07J 41/00, C07J 53/00, C07J 63/00, C07J 71/00

(54) **STEROIDSULFAMATE, VERFAHREN ZU IHRER HERSTELLUNG UND ANWENDUNG DERSELBEN**
STEROID SULFAMATES, METHOD FOR THE PRODUCTION AND USE THEREOF
SULFAMATE DE STEROIDE, PROCEDE DE PRODUCTION ET UTILISATION DE CETTE SUBSTANCE

(30) Priorität: 25.03.1997 DE 19712488
(43) Veröffentlichungstag der Anmeldung: 12.01.2000
(60) Teilanmeldung: 06016215.3
(73) Patentinhaber: Sterix Limited, Slough, Berkshire SL1 3XE (GB)
(72) Erfinder: KASCH, Helmut, D-07745 Jena (DE); SCHUMANN, Winfried, D-07745 Jena (DE); RÖMER, Johannes, D-01454 Grosserkmannsdorf (DE); STEINBACH, Jörg, D-01277 Dresden (DE)
(74) Vertreter: Alcock, David
(86) Internationale Anmeldenummer: PCT/DE1998/000813
(87) Internationale Veröffentlichungsnummer: WO 1998/042729

(56) Entgegenhaltungen:
- WO-A-93/05064
- WO-A-96/05216
- WO-A-96/05217
- SCHWARZ S ET AL: "Steroidsulfamate" ZEITSCHRIFT FUER CHEMIE, DEUTSCHER VERLAG FUER GRUNDSTOFFINDUSTRIE,, DE, vol. 14, no. 1, 1974, pages 15-16, XP002171269 ISSN: 0044-2402
- ELGER W ET AL: "Sulfamates of various estrogens are prodrugs with increased systemic and reduced hepatic estrogenicity at oral application." THE JOURNAL OF STEROID BIOCHEMISTRY AND MOLECULAR BIOLOGY. ENGLAND DEC 1995, vol. 55, no. 3-4, December 1995 (1995-12), pages 395-403, XP002268508 ISSN: 0960-0760
- ROEMER, J. ET AL: "Examinations of the reaction of 16,17-O-sulfuryl-16-epiestriol-3- sulfamate with [18F]fluoride" FORSCHUNGSZENTRUM ROSSENDORF E.V., [BERICHT] FZR (1997), FZR-200, 185-188, XP002268509
- ROEMER, J. ET AL: "Sulfamates of 3-hydroxy-estra-1-3-5(10)-triene derivatives" FORSCHUNGSZENTRUM ROSSENDORF E.V., [BERICHT] FZR (1997), FZR-165, 182-187, XP002268510

## Beschreibung

Die vorliegende Erfindung betrifft Steroide vom Gonantyp mit sulfatasehemmender und / oder estrogener Wirkung für die Anwendung in der pharmazeutischen Forschung und industrie.

Steroid-3-sulfamate mit einer Sulfamoyloxy-, Alkyl-, Cycloalkyl- oder Dialkylsulfamoyloxygruppe sind seit langem bekannt (DE 3376799,1998, Schwarz,S. Pharmazie 30 (1975) 17 - 21). Sie werden aufgrund ihrer besseren Bioverfügbarkeit und ihrer geringeren Metabolisierung bei der Leberpassage als Prodrugs für Estrogene in der Substitutionstherapie, in Form von Kombinationspräparaten bei der Kontrazeption und als Substanzen mit Scavenger-Eigenschaften eingesetzt.
An Steroiddisulfamaten (DE 2,336,431) kennt man lediglich das 1,3-Dialkylsulfamoyloxy-8α-estratrien-17-on, das ebenfalls eine estrogene Wirksamkeit aufweist, ferner gewisse ggf. substituierte 3,11- und 3,17-Disulfamoyloxy-estratriene, die insbesondere als hormonale Kontrazeptiva eingesetzt werden (WO 96/05216).
In jüngster Zeit wurde von M.J.Reed et al. [Biochemistry 34 (1995) 11508 - 11514: J.Steroid Biochem.Molec.Biol.57 (1996) 79 - 88] gefunden, dass von Estradiol- und Estron-3-sulfamaten eine starke Sulfatasehemmwirkung ausgeht. Sulfatasehemmer können zur Bekämpfung von estrogenabhängigen Tumoren eingesetzt werden. Indem sie die Freisetzung von Estradiol oder Estron aus endogenen Steroidkonjugaten, den entsprechenden Sulfaten, unterbinden. Es wurde später darauf hingewiesen, dass steroidale Sulfatasehemmer vom Typ des Estradiol- oder Estron-3-sulfamats als Sulfatasehemmer zur Bekämpfung von estrogenabhängigen Tumoren nur bedingt einsetzbar sind. Bei in vivo Experimenten weisen diese Verbindungen eine potenzierte estrogene Wirkung auf [Eiger W. et al. J.Ster.Biochem.Molec.Biol. 55 (1995) 395 - 403], die bei dieser indikation unerwünscht ist.

Über nichtsteroidale Sulfatasehemmer mit vergleichbar hoher Sulfatasehemmwirkung, entsprechend Estronsulfamat, wurde kürzlich berichtet (Li P.-K et al. J.Ster.Biochem.Molec. Biol. 59(1996) 41 - 48).
Eine völlige Unterdrückung der estrogenen Wirkung wurde sowohl bei den nichtsteroidalen als auch bei den steroidalen Sulfatasehemmern bei gleichbleibender oder verbesserter Sulfatasehemmwirkung bisher nicht beobachtet.

Die WO 93/05064 offenbart Steroid-3-monosulfamate, die eine Steroidsulfatasehemmwirkung aufweisen.

Es besteht deshalb ein Bedarf an Verbindungen, die die Verfügbarkeit von Estrogenen in hormonabhängigen Tumoren einschränkt bzw. unterbindet, wobei Sulfatasehemmer, die keine estrogene Wirkkomponente aufweisen, von interesse sind.
Weiter ist bekannt, dass die Estrogenität von Estradiol durch gezielte Substitution am D-Ring variiert werden kann. Die orale Wirksamkeit kann durch Substitution (Alkylierung oder Ethinierung) des zur 17-Hydroxygruppe geminal stehenden Protons entscheidend verbessert werden. Substitution des zur 17β-Hydroxygruppe vicinal stehenden Protons in 16-Stellung führt zu einer Verminderung der oralen Wirksamkeit Substitution durch Halogen oder Pseudohalogen führt dabei zu sogenannten gebremsten, d.h. schwach oder moderat wirksamen, Estrogenen.
Da bei 17-alkylierten oder ethinierten Estradiolderivaten bei Langzeitversuchen Lebertoxizität festgestellt wurde, besteht weiterhin ein interesse an hochwirksamen Estrogenen mit geringer Toxizität, um diese Verbindungen abzulösen.

Die Aufgabe wird durch die Bereitstellung neuer Steroidsulfamoyloxyderivate, die durch geeignete Sulfamoylierungsreaktionen hergestellt werden, gelöst.
Es wurde gefunden, dass bestimmte steroidale Sulfamoyloxyverbindungen mit mehr als einer Sulfamatgruppierung im Molekül, insbesondere solche, die an den für die estrogene Wirkung charakteristischen Positionen, einschliesslich Substituenten oder Seitenketten (z.B. in 7- und / oder 11-Stellung), die sich an der Peripherie des Steroidgerüstes befinden können, sulfamoyliert sind, eine deutliche Erhöhung der Sulfataseaktivität bei verminderter estrogener Wirkung aufweisen. Dabei kann die Estrogenität in einigen Fällen so stark herabgesetzt werden, dass sogar antiestrogene Effekte nachweisbar sind.
Erfindungsgemäß wurde gefunden, daß Verbindungen vom Typ des Estradiol-disulfamats und des 16-Halogen-estradioldisulfamats eine stärkere Sulfatasehemmung als alle bekannten Verbindungen aufweisen und darüberhinaus keine Estrogenität besitzen. Durch die Halogensubstitution in 16-Stellung wird die Sulfatasehemmwirkung im Vergleich zur unsubstituierten Verbindung noch verstärkt.
Auch einige Monosulfamate, sieht man einmal von den Ring A - Sulfamoyloxyverbindungen ab, weisen eine signifikante Sulfataseaktivität bei verminderter Estrogenität auf. Bei Anwesenheit von zwei und mehr derartiger pharmakophorer Gruppierungen in einem Molekül sind synergistische Effekte zu beobachten. Diese führen in Einzelfällen zu einer Vervielfachung der Wirkung im Vergleich zu den Standardverbindungen, was anhand der Di- und Trisulfamate nachgewiesen wurde.

Aufgrund der Beeinflussung der Estrogenbiosynthese, der direkten Sulfatasehemmung und anderer antiestrogener Effekte wird die Verfügbarkeit von Estrogenen eingeschränkt bzw. kann reguliert werden, was für die Tumortherapie estrogenabhängiger Tumore einerseits und die Diagnostik andererseits genutzt werden kann.

Die Wirkungsdissoziation der Sulfamoyloxyverbindungen kann in Abhängigkeit vom Substitutionsmuster gezielt verändert werden, was den Einsatz als Sulfatasehemmer einerseits und den als estrogener Komponente andererseits möglich macht. Mittels eines Estrogentranskriptionsassays lassen sich bei einigen Sulfamaten überraschenderweise in vitro potentielle estrogene Wirkungen nachweisen 3,17-Disulfamoyloxyverbindungen erweisen sich in diesem Test als inaktiv.

16-Substituierte 3-Mono-sulfamate, zeigen eine außergewöhnliche therapeutische Breite. Neben einer hohen Sulfataseaktivität zeichnen sich diese Verbindungen durch eine hohe Estrogenität aus. Diese ist, vergleicht man sie mit den bekannten stark wirksamen Estron- oder Estradiol-3-sulfamaten, überraschend hoch. Nach dem Stand der Technik sind 16-Halogen- oder Pseudohalogen-estradiole im Vergleich zu Estriol oder Estradiol weniger wirksam. Mit der Einführung des Sulfamatrestes in 3-Stellung wird die Estrogenität erheblich gesteigert. So ist 16α-Bromestradiol-3-sulfamat bei oraler Applikation in vivo 5-fach wirksamer als Estriol und dreimal stärker wirksam als Estradiol-3-sulfamat. Mit diesen Befunden stehen neue oral wirksame Estrogene für die Kontrazeption und die Hormon-Replacement-Therapie einerseits und für die Diagnostik bei Anwendung radioaktiver Spezies (PET) andererseits zur Verfügung.

In markierter Form, insbesondere mit kurzlebigen Isotopen, wie [¹⁸F], [⁷⁶Br] oder Tc etc., stellen die erfindungsgemäßen Verbindungen aufgrund ihrer Targetspezifität gegenüber der Steroidsulfatase aber auch Estrogenrezeptoren potentielle Marker zur Diagnostizierung von krankhaften, unter anderem krebsartigen, Geweben dar.

Bei den erfindungsgemäßen Verbindungen handelt es sich um äusserst wirksame Sulfatasehemmer, die allein oder in Kombination mit anderen Wirkkomponenten, z.B. Aromatasehemmern oder Antiestrogenen, für die Behandlung von Krankheiten, die auf eine Inhibition der Sulfatase oder von Estrogenen, z. B. zur Bekämpfung von hormonabhängigen Tumoren, geeignet sind.

Darüber hinaus besitzen einige der steroidalen Sulfamoyloxyverbindungen in Kombination mit einem Gestagen auch Bedeutung als Mittel für die Kontrazeption und für die Behandlung von klimakterischen Beschwerden.

Die erfindungsgemäßen Verbindungen der allgemeinen Formel I können in Form von pharmazeutischen Präparaten Verwendung finden. Die Herstellung der Präparate erfolgt nach an sich bekannten Methoden der Galenik durch Mischen mit organischen und / oder anorganischen inerten Trägermateriallen, welche für enterale, perkutane oder parenterale Applikationen geeignet sind.

Geeignete Dosierungen können routinemässig durch Ermittlung der Bioäquivalenz gegenüber einem bekannten Sulfatasehemmer oder einem bekannten Estrogen bestimmt werden. Die Dosierung der erfindungsgemassen Verbindungen beträgt 0,001 bis 200 mg pro Tag.

Bei den erfindungsgemäßen Sulfamoyloxyverbindungen handelt es sich um Steroide vom Gonantyp der Formel I, 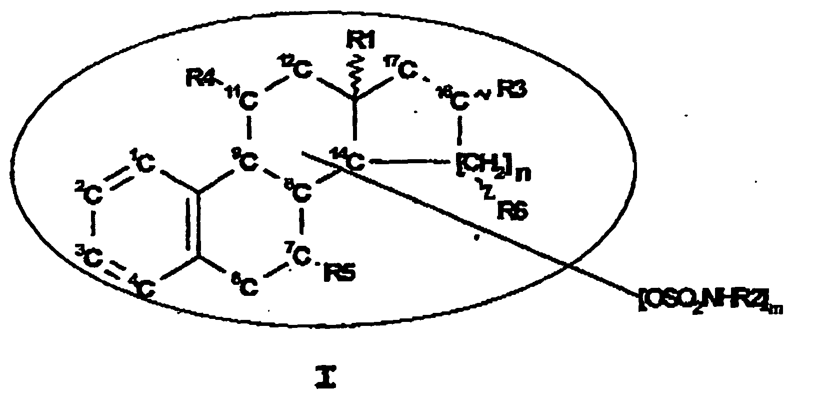 worin die C-Atome 2, 4, 6 und 12 unsubstituiert oder durch C₁-C₆ -Alkyloxy, C₁-C₄-Alkyloxy-C₁-C₄-alkyloxy, Hydroxy-C₁-C₄-alkyloxy, C₁-C₆₋Alkanoyloxy oder Tris-(C₁-C₄-Alkyl)-silyloxy oder Hydroxy, oder durch eine Ketogruppe -C(=O)- oder eine Form eines Ketals, Thioketals, Cyanhydrins, Cyansilylethers oder einer geminalen Hydroxyethinylgruppe geschütze Ketogruppierung substituiert sind,
n = 1 ist,
R₁ = für H, α- oder β-Methyl oder α- oder β-Ethyl steht,
R₂ = H, C₁ - C₅ - Alkyl, C₁ - C₃ -Alkyl mit anneliertem gesättigten Ring, Aryl-C₁-C₃-alkyl, C₁ - C₅- Alkanoyl, C₃- C₇-Cycloalkyl-carbonyl bedeutet,
R₃ = H, OH oder Halogen, C₁₋C₃-Alkyl, C₃- C₇-Cycloalkyl, 1',1'-Cycloalkyl oder Aryl-C₁-C₃-alkyl bedeutet,
R₄ = H, Aryl oder C₁ - C₁₂-Alkyl ist.
R₅ = H, C₁ - C₁₂ - Alkyl oder C₁- C₁₂ - Alkylaryl darstellt,
R₆ = H oder Halogen bedeutet oder eine Cyclopropano- oder Epoxidgruppierung mit α- oder β-Orientierung zwischen den C-Atomen 14 und 15 oder den C-Atomen 15 und 16 enthalten ist;
m = 2 ist; und,
die Sulfamoyloxygruppen -OSO₂NHR₂ an den C-Atomen 3 und 17 gebunden sind;
mit der Maßgabe, dass das Steroid nicht Oestra-1,3,5(10)-trien-3,17-β-diyl-3,17-sulfamat ist;
und Satze, insbesondere pharmazeutisch annehmbare Salze, davon.

1',1'-Cycloalkyl steht dabei für Spiro-C₃- C₆ -Alkyl oder -Alkenyl, Aryl-C₁-C₃- alkyl steht z.B. für Phenyl- C₁- C₃- alkyl wie Benzyl oder für Heteroar-C₁-C₃-alkyl, worin Heteroaryl z.B. für den Rest von Pyridin, Picolin, Lutidin, Collidin, Chinolin, Acridin, Pyridazin, Pyrimidin, Pyrazin, Triazin, Pterine, Pyrrol, lndol, Pyrazol, Imidazol, 1,2,3-Triazol, 1,2,4-Triazol, Tetrazol, Oxazol, Thiazol oder Thiodiazol steht, unter einem gesättigten annelierten Ring sind neben C₃- C₇ - Cycloalkyl hydrierte heterocyclische Ringe, wie Piperidin, Piperazin, Pyrrolin, Pyrolidin, Oxazolin, Oxazolidin, Thiazolin, Thiazolidin, Imidazolin oder Imidazolidin zu verstehen, und unter Aryl wird ein Heteroarylsubstituent obiger Bedeutung oder ein o-, m- oder p-substituierter Phenylrest verstanden (Substituenten eines solchen phenylrestes sind z.B. Halogen, C₁ - C₅ - Alkylthio, C₁ - C₅ - Alkyloxy, Hydroxy, C₁ - C₅ - Alkyl, Hydroxy- C₁ - C₅ - alkyl, C₁ - C₅ - Alkanoyl, CN, Amino, Mono- oder Di - C₁ - C₃ - alkylamino, Nitro oder CHO und Acetate und Oxime der Formel -CH(OR)(OR') und -CH=NOR davon, worin R und R' für H oder C₁ - C₅ - Alkyl steht oder R und R' zusammen für C₂ - C₄ - Alkylen stehen).

Vorzugsweise bedeutet dabei
R₁ = α- oder β-Methyl bzw. α- oder β-Ethyl,
R₃ = H, OH, Chlor, Brom, Fluor, N₃, CN, SCN oder SeCN.
R₂ in dem Sulfamoyloxyrest oder -resten (-OSO₂ NHR₂) H und / oder C₁ - C₅ - Alkyl,
C₁-C₃-Alkanoyl oderC₃-C₇-Cycloalkyl-carbonyl ist,
R₄ = H, Aryl oder C₁ - C₁₂- Alkyl,
R₅ = H, C₁ - C₁₂- Alkyl oder C₁- C₁₂ - Alkylaryl,
R₆ = H, Chlor, Brom oder Fluor,
und Salze davon.

In weiteren bevorzugten Verbindungen der Formel I bedeutet
R₁ = β-Methyl oder β-Ethyl
R₃ = Brom, [⁷⁶Br] Brom, Fluor, [¹⁸F] Fluor, [¹²⁵I]- oder [¹³¹I]- Iod oder Astatin,
R₅ = Brom, [⁷⁶Br] Brom, Fluor oder [¹⁸F] Fluor,
und R₂, R₄, R₅ und n haben die oben angegebenen Bedeutungen,
und Salze davon.

Eine weitere bevorzugte Gruppe von erfindungsgemäßen Verbindungen betrifft solche der Formel I, worin
R₅ = H oder Halogen, wie Chlor, Brom, [⁷⁶Br] Brom, Iod, Astatin, Fluor oder ¹⁸[F] Fluor, bedeutet
R₂ = H, C₁ - C₅ - Alkyl, C₁ - C₃ -Alkyl mit anneliertem gesättigten Ring oder Aryl-C₁- C₃₋alkyl ist,
R₄= Aryl oder C₁ - C₁₂-Alkyl bedeutet,
R₅ = C₁ - C₁₂ - Alkyl darstellt,
R₆ = Halogen, wie Chlor, Brom, [⁷⁶Br] Brom, [¹⁸F] Fluor, Fluor oder Astatin, bedeutet,
und Salze davon.

Besonders bevorzugte Verbindungen der Erfindung sind:
3,17β-Disulfamoyloxy-13β-ethyl-1,3,5(10)-gonatrien
3,17β-Disulfamoyloxy-13β-methyl-1,3,5(10),7(8)-gona-tetraen
3,17β-Disulfamoyloxy-13β-methyl-1,3,5(10)8,6-gona-pentaen
3,17β-Disulfamoyloxy-13β-methyl-1,3,5(10),8-gona-tetraen
3,17β-Disulfamoyloxy-13β-methyl-1,3,5(10),8,14-gona-pentaen
3,17β-Disulfamoyloxy-13β-methyl-1,3,5(10),8(14)-gona-tetraen
3,17β-Disulfamoyloxy-13β-methyl-1,3,5(10),9(11)-gona-tetraen
3,17β-Disulfamoyloxy-13β-ethyl-1,3,5(10),9(11)-gonatetraen
3,17β-Disulfamoyloxy-14β,15β-methylen-13β-methyl-1,3,5(10),8-gona-tetraen
3,17α-Disuffamoyloxy-14β,15β-methylen-13β-methyl-1,3,5(10),8-gona-tetraen
3,17β-Disutfamoyloxy-14α,15α-methylen-13β-methyl-1,3,5(10),8-gona-tetraen
3,17α-Disulfamoyloxy-14α,15α-methylen-13β-methyl-1,3,5(10),8-gona-tetraen
16α-Brom-3,17β-disulfamoyloxy-13β-methyl-1,3,5(10)-gonatrien
16α-Brom-3,17β-disulfamoyloxy-13β-ethyl-1,3,5(10)-gonatrien
16β-Brom-3,17β-disulfamoyloxy-13β-methyl-1,3,5(10)-gonatrien
16α-Chlor-3,17β-disulfamoyloxy-13β-methyl-1,3,5(10)-gonatrien
16α-Chlor-3,17β-disulfamoyloxy-13β-ethyl-1,3,5(10)-gonatrien
16β-Chlor-3,17β-disulfamoyloxy-13β-methyl-1,3,5(10)-gonatrien
3,17β-Disulfamoyloxy-16α-fluor-13β-methyl-1,3,5(10)-gonatrien
3,17β-Disulfamoyloxy-16α-fluor-13β-ethyl-1,3,5(10)-gonatrien
3,17α-Disulfamoyloxy-13β-methyl-1,3,5(10)-gona-trien.

Die erfindungsgemäßen Verbindungen werden zum Beispiel hergestellt, indem in an sich bekannter Weise Steroidalkohole der allgemeinen Formel II, 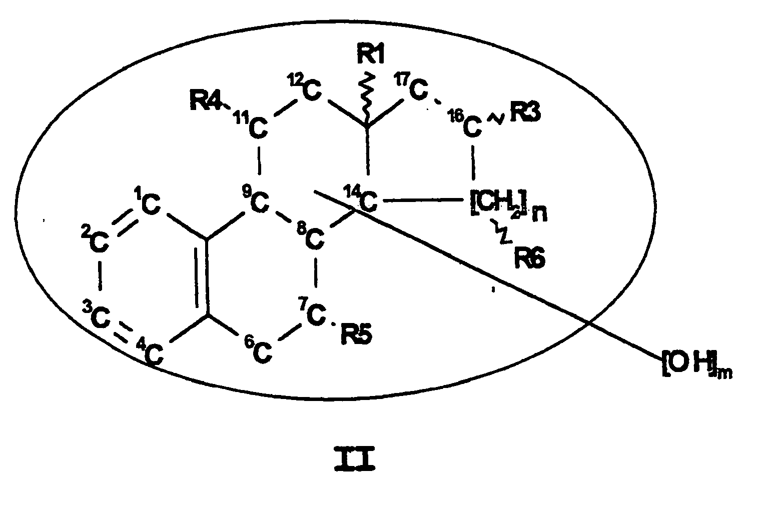 worin die Substituenten die oben angegebenen Bedeutungen haben,
- in einem geeigneten Lösungsmittel gelöst oder suspendiert werden,
- mit einer Base partiell bzw.quantitativ in Alkoholate überführt werden, was gegebenenfalls auch durch Phasentransferkatalyse realisiert wird,
- oder aber mit einem Puffer in Form eines tert. Amins, einer Pyridinbase oder eines wasserfreien Salzes versetzt werden,
und mit Amidosulfonsäurechloriden, welche N-alkyliert oder N-alkanoyliert sein können, zu den entsprechenden Amidosulfonaten umgesetzt werden.

Bevorzugt werden
- als Lösungsmittel
   halogenierte Kohlenwasserstoffe, wie Methylenchlorid oder Chloroform, Essigester, Tetrahydrofuran, Methyl-tert.butylether oder Ether, Acetonitril, DMF, DMSO, Benzol, Toluol oder Gemische davon verwendet.
- bei der Überführung in die Alkoholate als Basen
   NaH, CaH₂, Lithiumalkyle, LDA, Lithiumnaphthalid, Kalium-tert-butylat, KOH oder NaOH, gegebenenfalls in Kombination mit einem Phasentransfersalz verwendet,
- als Puffer
   Triethylamin, 2,6- oder 2,4,6-alkylierte Pyridinbasen, wasserfreies K₂CO₃ oder Na₂CO₃, letztere gegebenenfalls in Verbindung mit einen Phasentransfersalz oder einem Kronenether genutzt,
- die Sulfamoylierung
   mit Amidosulfonsäurechloriden, wie Sulfamoylchlorid, N-Alkyl- oder N-alkanoylamidosulfonsäurechloriden, gegebenenfalls verschiedener Art, schrittweise durchgeführt.

Bei der Reaktion wird für eine optimale Durchmischung, gegebenenfalls unter Kühlung, gesorgt, wobei vorteilhaft Ultraschall angewendet wird.

Die nachstehenden Beispiele dienen der näheren Erläuterung der Erfindung, ohne sie in irgendeiner Weise einzuschränken.

### Vergleichsbeispiel 1

### 16β,17β-Dihydroxy-3-sulfamoyloxy-13β-methyl-1,3,5(10)-gonatrien-16,17-sulfat

147 mg (0.373 mMol) 16β,17β-Dihydroxy-3-methoxymethyloxy-1,3,5(10)-gonatrien-16,17-sulfat werden in 5 ml Acetonitril gelöst und mit 45 µl HCL (1 M) versetzt. Man erhitzt das Reaktionsgemisch 10 Minuten auf 110° C unter Rühren, destilliert das Lösungsmittel ab und fügt bei Raumtemperatur 6 ml Methylenchlorid (ü.P₂O₅ destilliert), 700 mg wasserfreies Na₂CO₃ und 180 mg (1.56 mMol) Sulfamoylchlorid hinzu. Das Reaktionsgemisch wird kräftig gerührt, nach erfolgter Umsetzung (ca. 6 Std.) mit Wasser versetzt und mit Ether extrahiert. Nach dem Abdampfen des Lösungsmittels wird der Rückstand mit wenig Acetonitril aufgenommen und an einer HPLC-Säule (ET 125/8/4 Nucleosil 120 - 5C18 Macherey § Nagel) chromatographiert. Die Detektion erfolgt mit einem L - 4500 DAD bei einer Wellenlänge von 275 nm. Eluiert wird mit Acetonitril und die Substanz, die bei einer Rₜ von 7,3 Min. eluiert wird, im Vakuum eingeengt. Es wurden 34,7 mg (60 %) eines pulverförmigen Produktes isoliert, welches aus Acetonitril kristallisiert wurde.
F_{MeCN}.: 195 bis 198°C

| | | | | | |
|---|---|---|---|---|---|
| Elementaranalyse: | ber. | C: 50,4; | H: 5,4; | N: 3,3; | S: 14,9 |
| | gef. | C: 49,3; | H: 6,1; | N: 3,5; | S: 14,2 |

MS: m/z 429-0934 (M⁺),350.1180 (85%, M⁺ - HMSO₂), (50%, M⁺ - HNSO₂,-SO₃)
UV_{Max}[nm] : 270 (Acetonitril)
IR [cm⁻¹; KBr]: 3504, 3404, 3308, 1489, 1447, 1382, 1202, 991, 839, 702, 641, 540 ¹³C-NMR(ppm; CDCl₃): C(1) 127.1, C(2) 120.0, C(3) 149.3, C(4) 122.7, C(5) 138.7, C(10) 138.4, C(12) 37.4, C(13) 44.1, C(14) 47.9, C(15) 31.3, C(16) 82.8, C(17) 91.3, C(18) 13.0.

### Vergleichsbeipiel 2

### 3-Sulfamoyloxy-16,17-(2',2'-propylendioxy)-13β-methyl-1,3,5(10)-gonatrien

75 mg (0.23 mM) 16,17-(2',2'-Propylendioxy)-13β-methyl-1,3,5(10)-gonatrien-3β-ol werden in 5 ml Methylenchlorid gelöst. Nach Zugabe von 500 mg wasserfreiem Na₂CO₃ und 30 mg (0.26 mM) H₂NSO₂Cl wird die Lösung bei Raumtemperatur 60 Stunden kräftig gerührt. Während dieser Zeit werden zusätzliche Portionen des Reagenzes (8* 10 mg) hinzugefügt. Zur Aufarbeitung wird die Suspension in Wasser eingerührt und das Steroid mit Ether extrahiert. Nachdem die organische Phase mit Wasser neutral gewaschen ist, trocknet man mit Natriumsulfat und dampft das Lösungsmittel im Vakuum ab. Der verbleibende Rückstand wird in wenig Acetonitril gelöst und durch präparative HPLC (ET 125/8/4 Nucleosil 120-5C18 Macherey § Nagel) unter Verwendung von Acetonitril als Elutionsmittel (Detektion L 4500 DAD, 275 nm, Rₜ 0 8,7 Min.) aufgetrennt, wobei 26,3 mg (24,75 %) in Form eines weißen Pulvers nach Umkristallisation aus Acetonitril isoliert werden konnten.
F: 180 bis 185°C

| | | | | | |
|---|---|---|---|---|---|
| Elementaranalse: | ber. | C(61.9), | H(7.1), | N(3.4), | S(7.9), |
| | gef. | C(62.1), | H(6.9), | N(3.6), | S(7.9). |

MS (m/z): 407.2 (M⁺) ber. f. C₂₁H₂₉NO₅S
UV_{Max}[nm]: 275 (Acetonitril)
IR [cm⁻¹; KBr]: 3464, 3385, 1604, 1497, 1444, 1371, 1281, 1260, 1205, 1152, 1059, 863

### Beispiel 3

### 3,17β-Disulfamoyloxy-13β-methyl-1,3,5(10)-gonatrien

70 mg (0.26 mM) 13β-Methyl-1,3,5(10)-gonatrien-3,17β-diol, 500 mg K₂CO₃, 30 mg Bu₄NBr werden in 3 ml Methylenchlorid und 1 ml Essigester suspendiert und nach Zugabe von 90 mg (0.68 mM) H₂NSO₂Cl unter Wasserausschluss im Ultraschallbad bei 25 bis 35°C behandelt. Nach ca. 60 Minuten gibt man weitere 45 mg (0.34 mM) H₂NSO₂Cl hinzu und rührt nach 2 Stunden in Wasser ein. Nach Extraktion des Steroids mit Ether wird das Lösungsmittel im Vakuum abgedampft und der Rückstand an Kieselgel 60 chromatographiert. Elution mit Toluol/Aceton (10:1) ergibt 21 mg (19 %) des Disulfamats, welches aus Essigester / n-Hexan kristallisiert werden kann.
F.: 191 bis 194°C
UV_{Max}[nm]: 275 nm (Acetonitril)
IR [cm⁻¹; KBr]: 3266, 3302, 3338, 3390 (NH), 1540, 1368, 1322, 1184, 830, 871, 817 MS m/z: 333.1370 (M⁺ -H₂NSO₃H ber. f. C₁₆H₂₃O₃NS; ES⁺: 452,8 (M+Na): 469,0 (M+K) ber. f. C₁₈H₂₆N₂O₆S₂.

### Vergleichsbeispiel 4

### 17β-Sulfamoyloxy-13β-methyl-1,3,5(10)-gonatrien-3-ol

70 mg (0.26 mM) 13β-Methyl-1,3,5(10)-gonatrien-3,17β-diol und 500 mg Na₂CO₃ werden in 5 ml Methylenchlorid suspendiert und nach Zugabe von 100 mg (0,86 mM) H₂NSO₂Cl und nach 6 Stunden von weiteren 100 mg (0,86 mM) im Ultraschallbad unter Wasserausschluß 16 Stunden behandelt. Zur Aufarbeitung wird in Wasser eingerührt und das Steroid mit Ether extrahiert. Der nach dem Abdampfen des Lösungsmittels verbleibende Rückstand wird an Kieselgel 60 chromatographiert. Als Elutionsmittel dient ein Toluol / Aceton - Gemisch (7:1). Es werden 25 mg (27,7 %) des 17-Sulfamats isoliert, welches aus Toluol / Aceton oder Essigester / n-Hexan kristallisiert werden kann.
F.: 165 bis 170°C

| | | | | | |
|---|---|---|---|---|---|
| Elementaranalyse: | gef. | C: 59,0 | H: 6,6 | N: 4,0 | S: 8,6 |
| | ber. | C: 61,5 | H: 7,1 | N: 4,0 | S: 9.1 |

MS m/z: gef. 351.1539 (M⁺) ber. 351,1540 f. C₁₈H₂₅NO₄S
UV_{Max}[nm]: 284 (Acetonitril)
IR [cm⁻¹; KBr]: 3414, 3278 (NH), 1346, 1177, 964, 869, 586

### Vergleichsbeispiel 5

### 3-Methoxy-17β-sulfamoyloxy-13β-methyl-1,3,5(10)-gonatrien

500 mg (1,74 mM) 3-Methoxy-13β-methyl-1,3,5(10)-gonatrien-17β-ol, 500 mg K₂CO₃ und 40 mg Bu₄NBr werden in 10 ml Methylenchlorid suspendiert und nach Zugabe von 360 mg (3,1 mM) H₂NSO₂Cl so lange bei Raumtemperatur intensiv gerührt, bis alles Ausgangsmaterial verbraucht ist. Nach erfolgter Umsetzung wird in Wasser eingerührt und das Steroid mit Ether extrahiert. Nach dem Abdampfen des Lösungsmittels wird der verbleibende Rückstand aus Essigester / n-Hexan oder Methylenchlorid / n-Hexan kristallisiert, wobei 420 mg (66,3%) des 17-Sulfamats resultieren.
F._{Methylenchlorid/n-Hexan}: 159 bis 164°C
UV_{Max}[nm]: 285 (Acetonitril)
IR [cm⁻¹; CHCl₃]: 3445, 3344, 1603. 1567, 1546, 1496, 1376, 1252, 1245, 1181. 987, 972, 911
Rₜ (Lichrosorb 100 C18, Nr. 1621225): 7,09

### Vergleichsbeispiel 6

### 3-Methoxy-17β-sulfamoyloxy-13β-ethyl-1,3,5(10)-gonatrien

100 mg (0,33 mM) 3-Methoxy-13β-ethyl-1,3,5(10)-gonatrien-17β-ol, 500 mg K₂CO₃ und 40 mg Bu₄NBr werden in 3 ml Methylenchlorid und 1 ml Essigester suspendiert und nach Zugabe von 190 mg (1,64 mM) H₂NSO₂Cl so lange bei Raumtemperatur intensiv gerührt, bis alles Ausgangsmaterial verbraucht ist. Nach erfolgter Umsetzung wird in Wasser eingerührt und das Steroid mit Ether extrahiert. Nach dem Abdampfen des Lösungsmittels wird der verbleibende Rückstand an Kieselgel 60 chromatographiert. Als Elutionsmittel dient ein Methylenchlorid/Essigester-Gemisch (20:1), Man erhält 80 mg (71,4 %) des 17-Sulfamats, umkristallisierbar aus Methylenchlorid/n-Hexan.
F.: 178 bis 184°C
UV_{Max'} [nm]: 283 (Acetonitril)
IR [cm⁻¹; CHCl₃]: 3445, 3348, 1804, 1567, 1496, 1372, 1306, 1251, 989, 963, 915
MS ES⁻: 384.5 (M-H) ber.f. C₁₉H₂₇NO₄S 365,48

### Beispiel 7

### 16α-Brom-3,17β-disulfamoyloxy-13β-methyl-1,3,5(10)-gonatrien

### 16α-Brom-3-sulfamoyloxy-13β-methyl-1,3,5(10)-gonatrien-17β-ol

70 mg (0.2 mM) 16α-Brom-13β-methyl-1,3,5(10)-gonatrien-17β-diol, 500 mg K₂CO₃ und 40 mg Bu₄NBr werden in 3 ml Methylenchlorid und 1 ml Essigester suspendiert und nach Zugabe von 170 mg (1,47 mM) H₂NSO₂Cl 4 Stunden im Ultaschallbad behandelt. Zwecks Aufarbeitung wird die Suspension in Wasser eingerührt und das Steroid mit Ether extrahiert. Nach dem Abdampfen des Lösungsmittels Wird der Rückstand an Kieselgel 60 chromatographiert. Als Elutionsmittel dient ein Methylenchlorid Essigester-Gemisch (20:1). 10 mg der dünnschichtmäßig einheitlich erscheinenden polaren Fraktionen (60 mg) werden eingeengt und durch präparative HPLC (Säule RP 18, 2 ml/Min, Jasco) mit Acetonitril als Elutionsmittel aufgetrennt. Es werden 2 mg 3,17-Disulfamat (Rₜ 5,8) und 3,5 mg 3-Monosulfamat (Rₜ 6,36) erhalten.
F._{Disulfamat/Methylenchlorid.}: 190 bis 184°C
MS-ES⁻: 506,9 (509,1, Isotop 81) (M - H) ber. exakte Masse 508,03 f. C₁₈H₂₅BrN₂ O₆S₂
UV_{Max}[nm]: 270 nm (Acetonitril)
IR [cm⁻¹, KBr]: 3345, 3348 (NH), 1396, 1193, 917
Rₜ : 5,9 (RP 18, 2 ml/Min)
¹H-NMR [ppm; CD₃OD]: 7,32 d (1H), 7,05 qu (2H), 7.008 d (4H), 4.74 d (17α-H), 4,36 o (16β-H), 0,88 s (13-CH₃)
F._{3-Monosulfamat/Methylenchlorid/n-Hexan} : 94 bis 104°C
MS-ES⁻: 428,3 (430,2, Isotop 81) (M-H) ber. exakte Masse 429,0609 f. C₁₈H₂₄BrNO₄S UV_{Max}[nm]: 270 nm (Acetonitril)
IR [cm⁻¹, CHCl₃] : 3608 (OH), 3452. 3348 (NH). 1395, 1185 (SO₃), 911 (NS)
Rₜ: 6.36 (RP 18, 2 ml / Min)
¹H-NMR [ppm; CD₃OD]: 7,31 d (1H), 7,03 qu (2H), 7.01d (4H), 4.13 o (16β-H), 3,88 d (17α-H), 0,776 s (13-CH₃)

### Vergleichsbeispiel 8

### 16α-Brom-17β-sulfamoyloxy-13β-methyl-1,3,5(10)-gonatrien-3-ol

70 mg (0,2 mM) 16α-Brom-13β-methyl-1,3,5(10)-gonatrien-3,17β-diol, 500 mg K₂CO₂ und 40 mg Bu₄NBr werden in 3 ml Methylenchlorid und 1 ml Essigester suspendiert und nach Zugabe von 90mg (0,78 mM) H₂NSO₂Cl 2 Stunden im Ultaschallbad behandelt. Zwecks Aufarbeitung wird die Suspension in Wasser eingerührt und das Steroid mit Ether extrahiert. Nach dem Abdampfen des Lösungsmittels wird der Rückstand an Kieselgel 60 chromatographiert. Als Elutionsmittel dient ein Methylenchlorid / Essigester- Gemisch (20:1). Die Mittelfraktionen [R_{f}= 0,635 (CH₂Cl₂ Essigester 3:1)] werden vereinigt, eingeengt und durch präparative HPLC (Säule RP 18,2 ml/ Min, Jasco) mit Acetonitril als Elutionsmittel aufgetrennt. Die Fraktion enthält 2 Hauptkomponenten (Rₜ= 8,219 : Rₜ = 9.83 RP18 präp. Säule oder Rₜ= 6.306 u. 7,273 anal. Säule Spherisorb 100 C18 Nr. 1621225). Die Fraktion mit der polareren Komponente (Rₜ:8,219) wird abgetrennt und aus Methylenchlorid / n-Hexan kristallisiert.
F._{Methylenchlorid/n-Hexan}: 217 bis 220°C
UV_{Max}[nm]: 285
MS E⁻: 428.4 (490.4 Isotop 81; M-H) ber. Masse 429.06 f.C₁₈H₂₄BrNO₄

### Vergleichsbeispiel 9

### 3,16α,17β-Trisulfamoyloxy-13β-methyl-1,3,5(10)-gonatrien

100 mg (0,347 mM) 13β-Methyl-1,3,5(10)-gonatrien-3,16α,17β-triol, 500 mg K₂CO₃ und 50 mg Bu₄NBr werden in 3 ml Methylenchlorid und 1 ml Essigester suspendiert und nach Zugabe von 350 mg (3,02 mM) H₂NSO₂Cl 5 Stunden im Ultraschallbad behandelt. Zwecks Aufarbeitung wird die Suspension in Wasser eingerührt und das Steroid mit Ether extrahiert. Nach dem Abdampfen des Lösungsmittels wird der verbleibende Rückstand an Kieselgel 60 chromatographiert, Als Elutionsmittel dient ein Toluol / Aceton - Gemisch (7:1). Die polaren Fraktionen (Rₜ= 0.09 Gel. CH₂Cl₂ / Essigester 3:1) werden eingeengt und mittels Methylenchlorid zur Kristallisation gebracht.
F.: 115 bis 122°C
MS-ES⁻: 524.4 (M - H) her. Masse 525,0909 f. C₁₈H₂₇N₃O₉S₃
UV_{Max}[nm]: 270 (Acetonitril)
IR [cm⁻¹ ; KBr]: 3388, 3284, 1549, 1488, 1369, 1180, 1000,8, 939, 921, 554
Rₜ : 7,29 (RP 18,2ml / Min) oder Rₜ: 5.717 (Spherisorb 100 C18, 0,5 ml/ Min)
¹H-NMR [ppm CD₃OD]: 7,32 d (1H), 7.05 qu (2H), 7,01 d (4H), 5,01 o (16β-H), 4.56 d (17α-H), 0,82 (13-CH₃)

### Vergleichsbeispiel 10

### 16α-Fluor-17β-sulfamoyloxy-13β-methyl-1,3,5(10)-gonatrien-3-ol

70 mg (0,24 mM) 16α-Fluor--13β-methyl-1,3,5(10)-gonatrien-3,17β-of und 500 mg K₂CO₃ werden in 3 ml Methylenchlorid und 1 ml Essigester suspendiert und nach Zugabe von 100 mg (0,87 mM) H₂NSO₂Cl 8 Stunden bei Raumtemperatur im Ultraschallbad behandelt. Anschließend wird in Wasser eingerührt und das Steroid mit Ether extrahiert. Nach dem Abdampfen des Lösungsmittels wird der verbleibende Rückstand an Kieselgel 60 chromatographiert. Als Elutionsmittel dient ein Methylenchlorid / Essigester-Gemisch (20:1). Man erhält 21 mg (23,6 %) des 17-Sulfamats, umkristallisierbar aus Methylenchlorid / n-Hexan.
F.: 230 bis 236°C
MS - ES⁻ : 368.5 (M-H) ber. Masse 369,45 f. C₁₈H₂₄NO₄S
UV_{Max}[nm]: 282 (Acetonitril)
IR [cm⁻¹; KBr]: 3268, 3375, 3552, 1607, 1578, 1552, 1493, 1440, 1362, 1178, 1000. 929, 850
Rₜ : 7,99 (RP18, 2ml/Min) oder Rₜ: 6,149 (Spherisorb 100 C18, 0,5 ml/Min. Acetonitril)

### Beispiel 11

### 3,17α-Disulfamoyloxy-13β-methyl-1,3,5(10)-gonatrien

### 3-Sulfamoyloxy-13β-methyl-1,3,5(10)-gonatrien-17α-ol

62 mg (0,19 mM) 13β-Methyl-1,3,5(10)-gonatrien-3,17α-diol werden in 10 ml Acetonitril in der Wärme gelöst und mit 500 mg Na₂CO₃ (wasserfrei) versetzt. Nach dem Erkalten der Lösung werden 30mg(0.26mM) H₂NSO₂Cl zugegeben und anschließend kräftig gerührt. In Abständen von 1 Stunde werden 9 Portionen H₂NSO₂Cl zu je 10 mg zusätzlich hinzugefügt. Zwecks Aufarbeitung wird nach 9 Stunden in verdünnte Salzsäurelösung eingerührt und das Steroid mit Ether extrahiert. Der nach dem Einengen der Extrakte verbleibende Rückstand wird mit 4 ml eines Acetonitril/Ethanol-Gemisches (3:1) aufgenommen und durch präparative HPLC (ET 125/8/4 Nucleosil 120-5C18 Macherey § Nagel, RT 7,0) unter Verwendung von Acetonitril als Elutionsmittel aufgetrennt. Nach dem Einengen der Fraktion mit der Rₜ = 7.O (R_{f}= 0.2; Gel Benzol:Aceton = 4:1) bzw. Rₜ= 8,1 (R_{f} = 0,22 Gel Benzol/Aceton 4:1) werden 28,9 mg des 3,17α-Disulfamats (35,4 %) und 9.8 mg (14,6 %) des 3 Monosulfamats jeweils in Form von weißen Pulvern isoliert.
F._{Disulfamat}:170 bis 172°C
MS- ES⁻: 429,4 (M - H) ber. Masse 430.53 f. C₁₈H₂₆ N₂O₆S₂; ES⁺ : 883 (2 M + Na)
UV_{Max}[nm]: 270 (Acetonitril)
IR [cm⁻¹; KBr]: 3396, 3336, 3264, 1601, 1547, 1489, 1372, 1327, 1184, 930
F._{3-Monosulfamat}:180 bis 189°C
MS m/z: 351,2 (M⁺) ber. 351, 15 f. C₁₈H₂₅NO₄S: 333,2 (M⁺- H₂O) ber.f. C₁₈H₂₃O₃NS
UV_{Max}[nm]: 269 (Acetonitril)
IR [cm⁻¹ ; KBr]: 3550, 3370, 3235, 3185, 1602, 1571, 1490, 1381, 1274, 1211, 1179,
1145, 923, 813, 720, 619, 556, 543

### Vergleichsbeispiel 12

### 17α-Sulfamoyloxy-13β-methyl-1,3,5(10)-gonatrien-3-ol

136 mg (0.5mM) 13β-Methyl-1,3,5(10)-gonatrien-3,17α-diol werden in 14 ml Acetonitril in der Wärme gelöst und mit 500 mg Na₂CO₃ (wasserfrei) versetzt. Nach dem Erkalten der Lösung werden 100 mg (0.86 mM) H₂NSO₂Cl zugegeben und anschließend kräftig gerührt.
Zwecks Aufarbeitung wird nach 90 Minuten in verdünnte Salzsäurelösung eingerührt und das Steroid mit Ether extrahiert Der nach dem Einengen der Extrakte verbleibende Rückstand wird mit 4 ml eines Acetonitril / Ethanol- Gemisches (3:1) aufgenommen und durch präparative HPLC (ET 125/8/4 Nucleosil 120-5C18 Macherey § Nagel, RT 7.0) unter Verwendung von Acetonitril / Wasser (19: [HK1]1) als Elutionsmittel aufgetrennt. Nach dem Einengen der Fraktion mit der Rₜ = 7,55 (R_{f}= 0.55, Gel, Benzol / Aceton = 4:1) wurden 21,9 mg (12,5 %) des 17α-Sulfamats in Form eines weißen Pulvers isoliert.
F.: 114 bis 120°C
MS: m/z : 294,2 [M⁺ - 97 (HO₃SNH₂)] ber. f. C₁₈H₂₂O entspricht C₁₈H₂₅ NO₄S (351.15) ES⁻: 350,4 (M-H)
UV_{Max}[nm]:283 (Acetonitril)
IR [cm⁻¹; KBr]: 3370, 3150, 1606, 1563, 1495, 1446, 1298, 1236, 1065, 685, 545

### Beispiel 13

### 3,17β-Disulfamoyloxy-13β-ethyl-1,3,5(10)-gonatrien

100 mg (0,35 mM) 13β-Ethyl-1,3,5(10)-gonatrien-3, 17β-diol, 500 mg K₂CO₃ und 40 mg Bu₄NBr werden in 2 ml Methylenchlorid und 2 ml Essigester suspendiert und nach Zugabe von 260 mg (2.25 mM) H₂NSO₂Cl 2,5 Stunden im Ultraschallbad behandelt. Zwecks Aufarbeitung wird die Suspension in Wasser eingerührt und das Steroid mit Ether extrahiert. Nach dem Abdampfen des Lösungsmittels wird der verbleibende Rückstand an Kieselgel 60 chromatographiert. Als Elutionsmittel dient ein Toluol / Aceton - Gemisch (10:1). Aus den polaren Fraktionen werden 31 mg (20%) des Disulfamats isoliert, welches aus Methylenchlorid / n-Hexan umkristallisiert werden kann.
F.: 173 bis 178°C
MS ES⁺ : 467.0 (M+Na), 911,9 (2M+Na); ES⁻; 443,5 (M-H), 587 (2M-H)
UV_{Max}[nm]: 270 (Acetonitril)
IR [cm⁻¹, KBr]: 3392, 3280, 1603, 1549, 1488, 1383, 1184, 1179, 1138, 921, 551
Rₜ = 5,955 (Lichrosorb 100 C18, 0.5 ml / Min) bzw. Rₜ = 7.67 (RP18, 2 ml / Min)
¹H-NMR [ppm CD₃OD]: 7,31 d (1H). 7.04 qu (2H), 7.01 d (4H), 4.64 qu (17α-H), 1,00 t (13-Ethyl)

### Vergleichsbeispiel 14

### 17β-Sulfamoyloxy-13β-ethyl-1,3,5(10)-gonatrien-3-ol

50 mg (0.17 mM) 13β-Ethyl-1,3,5(10)-gonatrien-3,17β-diol und 500 mg K₂CO₃ und 40 mg Bu₄NBr werden in 2 ml Methylenchlorid und 2 ml Essigester suspendiert und nach Zugabe von 115 mg (1 mM) H₂NSO₂Cl 6 Stunden im Ultraschallbad behandelt. Zwecks Aufarbeitung wird die suspension in Wasser eingerührt und das Steroid mit Ether extrahiert. Nach dem Abdampfen des Lösungsmittels wird der verbleibende Rückstand an Kieselgel 60 chromatographiert. Als Elutionsmittel dient ein Toluol/ Aceton - Gemisch (10:1). Die Fraktionen mit einem R_{f} = 0,41 (Gel, Toluol / Aceton 4:1) bzw. R_{f} = 0,62 (Gel, CH₂Cl₂ / Essigester 3:1), Rₜ = 8,506 (RP 18) werden eingeengt (15 mg = 23.8 %) und aus Methylenchlorid / n-Hexan kristallisiert.
F.: 174 bis 176°C
MS ES⁻: 364.5 (M - H), 729,5 (2M -H)
UV_{Max}[nm]: 285 (Acetonitril)
IR [cm⁻¹ ; CHCl₃]: 3592, 3445, 3348, 1608, 1545, 1497, 1443, 1374, 1184, 1065, 917
Rₜ = 8.506 (RP18, 2 ml /Min)

### Beispiel 15

### 3,17β-Disulfamoyloxy-16α-fluor-13β-methyl-1,3,5(10)-gonatrien

15 mg 16α-Fluor-13β-methyl-1,3,5(10)-estratrien-3,17β-diol werden in 1 ml DMF gelöst und unter Kühlung mit 45 mg H₂NSO₂Cl unter inertgas und Rühren versetzt. Nach zwei Stunden wird zwecks Aufarbeitung in eine gesättigte Bicarbonatlösung eingerührt und das Steroid mit Methylenchlorid extrahiert. Der nach dem Einengen der Methylenchloridextrakte verbleibende Rückstand wird an Kiesel chromatographiert. Nach Elution mit Methylenchlorid /Essigester (6:1) werden die Fraktionen mit dem R_{F} = 0,6 [(Kieselgel; Methylenchlorid / Essigester 3:1) (Rₜ = 5,833; RP 18. Acetonitril)] eingeengt und aus Methylenchlorid kristallisiert.
F.: 178 bis 184°C
MS ES⁻: 447,4 (M - H), 894.7 (2M -H), 671 (3M⁻-H)
MS ES⁺: 471,2 (M + Na), 919,2 (2M + Na), 695.6 (3M + 2 Na)
Rₜ = 5,833 (RP 18; 0,5 ml/Min. CH₃CN)

### Beispiel 16

In analoger Weise (entsprechend den Beispielen 1-15) können
3,17β-Disulfamoyloxy-13β-methyl-D-homo-1,3,5(10)-gonatrien
3,17β-Disulfamoyloxy-13β-methyl-8α-D-homo-1,3,5(10)-gonatrien
3,17β-Disulfamoyloxy-13β-methyl-1,3,5(10),7(8)-gona-tetraen
3,17β-Disulfamoyloxy-13β-meihyl-1,3,5(10)8.6-gona-pentaenn
3.17β-Disulfamoyloxy-13β-methyl-1,3,5(10),8-gona-tetraen
3,17β-Disulfamoyloxy-13β-methyl-1,3,5(10),8,14-gona-pentaen
3,17β-Disulfamoyloxy-13β-methyl-1,3,5(10),8(14)-gona-tetraen
3,17β-Disulfamoyloxy-13β-methyl-1,3,5(10),9(11)-gona-tetraen
3,17β-Disulfamoyloxy-13β-ethyl-1,3,5(10),9(11)-gonatetraen
3,17β-Disulfamoyloxy-14β,15β-methylen-13β-methyl-1,3,5(10),8-gona-tatraen
3,17α-Disulfamoyloxy-14β,15β-methylen-13β-methyl-1,3,5(10),8-gona-tetraen
3,17β-Disulfamoyloxy-14α,15α-methylen-13β-methyl-1,3,5(10),8-gona-tetraen
3,17α-Disulfamoyloxy-14α,15α-methylen-13β-methyl-1,3,5(10),8-gona-tetraen
16α-Brom-3,17β-disulfamoyloxy-13β-ethyl-1,3,5(10)-gonatrien
16β-Brom-3,17β-disulfamoyloxy-13β-methyl-1,3,5(10)-gonatrien
16α-Chlor-3,17β-disulfamoyloxy-13β-methyl-1,3,5(10)-gonatrien
16α-Chlor-3,17β-disulfamoyloxy-13β-ethyl-1,3,5(10)-gonatrien
16β-Chlor-3,17β-disulfamoyloxy-13β-methyl-1,3,5(10)-gonatrien
3,17α-Disulfamoyloxy-13β-methyl-1,3,5(10)-gona-trien
hergestellt werden.

## Patentansprüche

1. Steroide des Gonantyps der allgemeinen Formel I 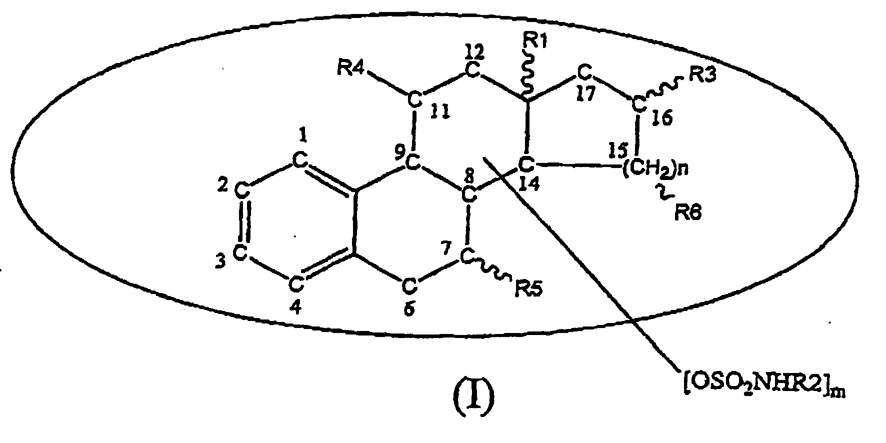 worin die C-Atome 2, 4, 6, und 12 unsubstituiert oder durch C₁₋₅-Alkyloxy, C₁₋₄-Alkyloxy-C₁₋₄-alkyloxy, Hydroxy-C₁₋₄-alkyloxy, C₁₋₆₋Alkanoyloxy oder Tris-(C₁₋₄-alkyl)-silyloxy oder Hydroxy substituiert sind oder eine Ketogruppierung (=O) oder eine Ketogruppierung, geschützt in Form eines Ketal-, Thioketal-, Cyanohydrin-, Cyanosilylethers oder einer geminalen Hydroxyethinylgruppe, anstelle einer sek. Hydroxygruppe vorhanden sein kann,
n 1 bedeutet,
R₂ α-Methyl, β-Methyl, α-Ethyl, β-Ethyl oder H ist,
R₂ H, C₁₋₅-Alkyl, C₁₋₃-Alkyl mit einem annellierten gesättigten Ring, Aryl-C₁₋₃-alkyl, C₁₋₅-Alkanoyl oder C₃₋₇-Cycloalkyl-carbonyl,
R₃ H, OH, Halogen, Pseudohalogen, C₁₋₃-Alkyl, C₃₋₇-Cycloalkyl, 1',1'-Cycloalkyl oder Aryl-C₁₋₃-alkyl,
R₄ H, Aryl oder C₁₋₁₂-Alkyl,
R₅ H, C₁₋₁₂-Alkyl oder C₁₋₁₂-Alkylaryl,
R₆ H oder Halogen oder ein Cyclopropan- oder Epoxid-Gruppierung mit α- oder β-Orientierung zwischen den C-Atomen 14 und 15 oder den C-Atomen 15 und 16 und
m 2 bedeuten,
die Sulfamoyloxygruppen -OSO₂NHR₂ mit den C-Atomen 3 und 17 verknüpft sind, mit der Maßgabe, dass das Steroid nicht Oestra-1,3,5(10)-trien-3,17-β-diyl-3,17-sulfamat ist,
und deren Salze.

2. Verbindungen der Formel I nach Anspruch 1, worin
R₂ H, α- oder β-Methyl oder β-Ethyl bedeutet,
R₃ H oder Halogen ist,
R₂ H, C₁₋₅ -Alkyl, C₁₋₇-Alkyl mit annelliertem gesättigtem Ring oder Aryl-C₁₋₃-alkyl bedeutet,
R₄ Aryl oder C₁₋₂₂-Alkyl,
R₅ C₁₋₁₂-Alkyl und
R₆ Halogen bedeuten,
und ihre Salze.

3. Verbindungen der Formel I nach Anspruch 1, worin
R₁ α- oder β-Methyl oder α- oder β-Ethyl,
R₃ H, OH, Chlor, Brom, Fluor, N₃, CN, SCN oder SeCN,
R₂ in den Sulfamoyloxyresten (-OSO₂NHR₂) H und/oder C₁₋₅-Alkyl, C₁₋₅-Alkanoyl oder C₃₋₇₋Cycloalkyl-carbonyl, und
R₆ H, Chlor, Brom oder Fluor Fluor bedeuten,
und ihre Salze.

4. Verbindungen der Formel I nach Anspruch 1, worin
R₂ β-Methyl oder β-Ethyl,
R₃ Brom, [⁷⁶Br]-Brom, Fluor, [¹⁸F]-Fluor, [²²⁵I]- oder [²³¹I]-Iod oder Astatin und
P₆ Brom, [⁷⁶Br]-Brom, Fluor oder [¹⁸F1]-Fluor bedeuten,
und R₂, R₄, R₃ und n die in Anspruch 3 angegebenen Bedeutungen haben, und
die Salze davon.

5. Verbindung der Formel I nach Anspruch 1, ausgewählt aus jeweils:
3,17β-Disulfamoyloxy-13β-ethyl-1,3,5(10)-gonatrien
3,17β-Disulfamoyloxy-13β-methyl-1,3,5(10),7(8)-gona-tetraen
3,17β-Disulfamoyloxy-13β-methyl-1,3,5(10)8,6-gona-pentaen
3,17β-Disulfamoyloxy-13β-methyl-1,3,5(10),8-gona-tetraen
3,17β-Disulfamoyloxy-13β-methyl-1,3,5(10),8,14-gona-pentaen
3,17β-Disulfamoyloxy-13β-methyl-1,3,5(10),8(14)-gona-tetraen
3,17β-Disulfamoyloxy-13β-methyl-1,3,5(10),9(11)-gona-tetraen
3,17β-Disutfamoyloxy-13β-ethyl-1,3,5(10),9(11)-gonatetraen
3,17β-Disulfamoyloxy-14β,15β-methylen-13β-methyl-1,3,5(10),8-gona-tetraen
3,17α-Disulfamoyloxy-14β,15β-methylen-13β-methyl-1,3,5(10),8-gona-tetraen
3,17β-Disulfamoyloxy-14α,15α-methylen-13β-methyl-1,3,5(10),8-gona-tetraen
3,17α-Disulfamoyloxy-14α,15α-methylen-13β-methyl-1,3,5(10),8-gona-tetraen
16α-Brom-3,17β-disulfamoyloxy-13β-methyl-1,3,5(10)-gonatrien
16α-Brom-3,17β-disulfamoyloxy-13β-ethyl-1,3,5(10)-gonatrien
16β-Brom-3,17β-disulfamoyloxy-13β-methyl-1,3,5(10)-gonatrien
16α-Chlor-3,17β-disulfamoyloxy-13β-methyl-1,3,5(10)-gonatrien
16α-Chlor-3,17β-disulfamoyloxy-13β-ethyl-1,3,5(10)-gonatrien
16β-Chlor-3,17β-disulfamoyloxy-13β-methyl-1,3,5(10)-gonaVien
3,17β-Disulfamoyloxy-16α-fluor-13β-methyl-1,3,5(10)-gonatrien
3,17β-Disulfamoyloxy-16α-fluor-13β-ethyl-1,3,5(10)-gonatrien
3,17α-Disulfamoyloxy-13β-methyl-1,3,5(10)-gona-trien
und ihre Salze.

6. Pharmazeutisches Präparat, enthaltend wenigstens eine Verbindung der Formel I nach Anspruch 1 sowie einen pharmazeutisch verträglichen Träger.

7. Verbindung der Formel I nach Anspruch 1 für therapeutische Zwecke.

8. Verwendung der Verbindung der Formel I nach Anspruch 1 zur Herstellung eines Medikaments für die Behandlung von hormonabhängigen Tumoren.

9. Verwendung von Verbindungen der Formel I nach Anspruch 1 zur Herstellung eines diagnostischen Mittels.

## Claims

1. Steroids of gonan type of general formula I, 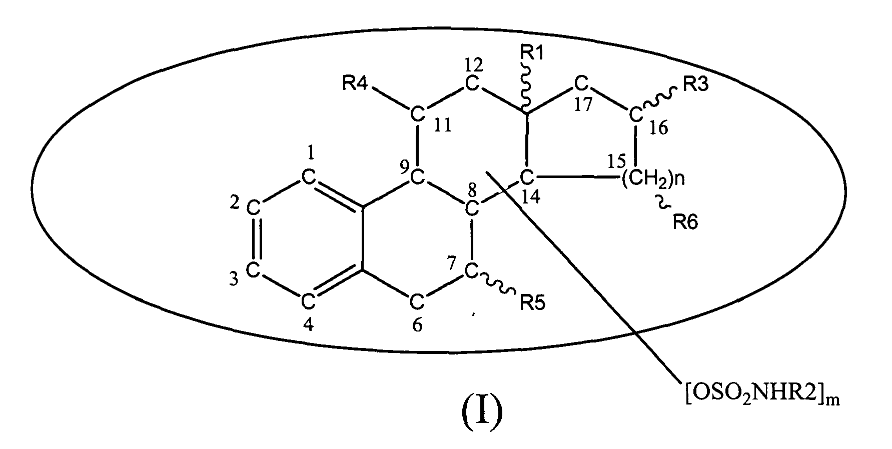 wherein the C atoms 2, 4, 6 and 12 are unsubstituted or substituted by C₁ - C₆ -alkyloxy, C₁ - C₄ -alkyloxy-C₁ - C₄ -alkyloxy, hydroxy-C₁ - C₄ - alkyloxy, C₁ - C₆-alkanoyloxy or tris- (C₁ - C₄-alkyl) - silyloxy or hydroxy, or a keto grouping (=O) or a keto grouping protected in the form of a ketal, thioketal, cyanohydrin, cyanosilyl ether or a geminal hydroxyethinyl group, can be present in the place of a secondary hydroxyl group,
n is 1,
R₁ is α-methyl, β-methyl, α-ethyl, β-ethyl or H;
R₂ is H, C₁ - C₅ -alkyl, C₁ - C₃-alkyl with annelated saturated ring, aryl-C₁-C₃-alkyl, C₁ - C₅ - alkanoyl, C₃- C₇-cycloalkyl-carbonyl,
R₃ is H, OH, halogen, pseudohalogen, C₁ - C₃-alkyl, C₃ - C₇cycloalkyl, 1',1'-cycloalkyl or aryl-C₁- C₃ -alkyl,
R₄ is H, aryl or C₁ - C₁₂-alkyl,
R₅ is H, C₁ - C₁₂ - alkyl or C₁ - C₁₂ - alkylaryl,
R₆ is H or halogen or a cyclopropane grouping or epoxide grouping with α or β orientation is provided between C atoms 14 and 15 or C atoms 15 and 16;
m is 2; and
the sulfamoyloxy groups -OSO₂NHR₂ are attached to C atoms 3 and 17;
provided that the steroid is not oestra-1,3,5(10)-triene-3,17-β-diyl-3,17-sulphamate;
and salts thereof.

2. Compounds of Formula 1 according to Claim 1, wherein
R₁ is H, α - or β-methyl or β-ethyl,
R₃ is H or halogen,
R₂ is H, C₁ - C₅-alkyl, C₁ - C₃-alkyl with annelated saturated ring or aryl-C₁ - C₃-alkyl,
R₄ is aryl or C₁ - C₁₂ -alkyl,
R₅ is C₁ - C₁₂- alkyl,
R₆ is halogen,
and salts thereof.

3. Compounds of formula I according to Claim 1, wherein
R₁ is α - or β-methyl, or α - or β-ethyl,
R₃ is H, OH, chlorine, bromine, fluorine, N₃, CN, SCN, or SeCN,
R₂ in the sulfamoyloxy residues (-OSO₂ NHR₂) is H and/or C₁ - C₅ - alkyl, C₁ - C₅ - alkanoyl or C₃ - C₇ -cycloalkyl-carbonyl,
R₆ is H, chlorine, bromine or fluorine,
and salts thereof.

4. Compounds of Formula I according to Claim 1, wherein
R₁ is β-methyl or β-ethyl
R₃ is bromine, [⁷⁶Br] bromine, fluorine, [¹⁸F] fluorine, [¹²⁵I]- or [¹³¹I]-iodine or astatine,
R₆ is bromine, [⁷⁶Br] bromine, fluorine or [¹⁸F] fluorine,
and R₂, R₄, R₅ and n have the meanings given in Claim 3,
and salts thereof.

5. A compound of formula I according to Claim 1, selected from:
3,17β-disulfamoyloxy-13β-ethyl-1,3,5(10)-gonatriene
3,17β-disulfamoyloxy-13β-methyl-1,3,5(10),7(8)-gona-tetraene
3,17β-disulfamoyloxy-13β-methyl-1,3,5(10)8,6-gona-pentaene
3,17β-disulfamoyloxy-13β-methyl-1,3,5(10),8-gona-tetraene
3,17β-disulfamoyloxy-13β-methyl-1,3,5(10),8,(14)-gona-pentaene
3,17β-disulfamoyloxy-13β-methyl-1,3,5(10),8(14)-gona-tetraene
3,17β-disulfamoyloxy-13β-methyl-1,3,5(10),9(11)-gona-tetraene
3,17β-disulfamoyloxy-13β-ethyl,1,3,5(10),9(11)-gonatetraene
3,17β-disulfamoyloxy-14β,15β-methylene-13β-methyl-1,3,5(10),8-gona-tetraene
3,17α-disulfamoyloxy-14β,15β-methylene-13β-methyl-1,3,5(10),8-gona-tetraene
3,17β-disulfamoyloxy-14α,15α-methylene-13β-methyl-1,3,5(10),8-gona-tetraene
3,17α-disulfamoyloxy-14α,15α-methylene-13β-methyl-1,3,5(10),8-gona-tetraene
16α-bromine-3,17β-disulfamoyloxy-13β-methyl-1,3,5(10)-gonatriene
16α-bromine-3,17β-disulfamoyloxy-13β-ethyl-1,3,5(10)-gonatriene
16β-bromine-3,17β-disulfamoyloxy-13β-methyl-1,3,5(10)-gonatriene
16α-chlorine-3,17β-disulfamoyloxy-13β-methyl-1,3,5(10)-gonatriene
16α-chlorine-3,17β-disulfamoyloxy-13β-ethyl-1,3,5(10)-gonatriene
16β-chlorine-3,17β-disulfamoyloxy-13β-methyl-1,3,5(10)-gonatriene
3,17β-disulfamoyloxy-16α-fluorine-13β-methyl-1,3,5(10)-gonatriene
3,17β-disulfamoyloxy-16α-fluorine-13β-ethyl-1,3,5(10)-gonatriene
3,17α-disulfamoyloxy-13β-methyl-1,3,5(10)-gona-triene
and salts thereof.

6. A pharmaceutical preparation containing at least one compound of formula I according to Claim 1, as well as a pharmaceutically acceptable carrier.

7. A compound of formula I as claimed in Claim 1 for use in therapy.

8. Use of compounds of formula I according to Claim 1 in the preparation of a medicament for the treatment of hormone-dependent tumours.

9. Use of compounds of formula I according to Claim 1 in the preparation of a diagnostic agent.

## Revendications

1. Stéroïdes du type gonane de formule générale I 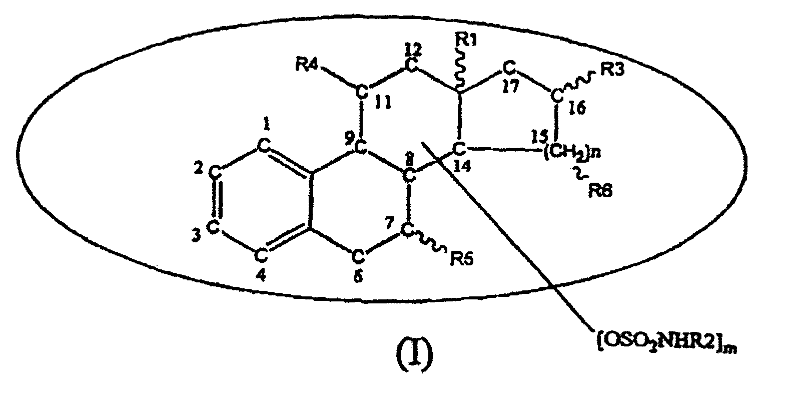 dans laquelle les atomes de carbone 2, 4, 6 et 12 sont non substitués ou sont substitués par un reste alkyloxy en C₁ à C₆, (alkyloxy en C₁ à C₄)-(alkyloxy en C₁ à C₄), hydroxy-(alkyloxy en C₁ à C₄), alcanoyloxy en C₁ à C₄ ou tris(alkyle en C₁ à C₄)silyloxy ou par un groupe hydroxy, ou bien un groupement céto (=O) ou un groupement céto protégé sous forme d'un éther de cétal, de thiocétal, de cyanhydrine ou de cyanosilyle ou d'un groupe hydroxyéthynyle géminal peut être présent à la place d'un groupe hydroxy secondaire,
n est égal à 1,
R₁ représente un reste α-méthyle, β-méthyle, α-éthyle, β-éthyle ou H,
R₂ représente H, un reste alkyle en C₁ à C₅, alkyle en C₁ à C₃ avec un noyau saturé condensé, aryl-(alkyle en C₁ à C₃), alcanoyle en C₁ à C₅ ou (cycloalkyle en C₅ à C₇) carbonyle,
R₃ représente H, un groupe OH, un halogène, un pseudo-halogène, un reste alkyle en C₁ à C₃, cycloalkyle en C₃ à C₇, 1',1'-cycloalkyle ou aryl-(alkyle en C₁ à C₃),
R₄ représente H, un reste aryle ou un reste alkyle en C₁ à C₁₂,
R₅ représente H, un reste alkyle en C₁ à C₁₂ ou (alkyle en C₁ à C₁₂) aryle,
R₆ représente H ou un halogène ou un groupement cyclopropane ou époxyde, avec orientation en α ou β entre les atomes de carbone 14 et 15 ou entre les atomes de carbone 15 et 16 et
m est égal à 2,
les groupes sulfamoyloxy -OSO₂NHR₂ sont liés aux atomes de carbone 3 et 17, sous réserve que le stéroïde ne soit pas le 3,17-sulfamate d'oestra-1,3,5(10)-triène-3,17-β-diyle,
et leurs sels.

2. Composés de formule (I) suivant la revendication 1, dans laquelle
R₁ représente H, un reste α-méthyle, β-méthyle ou β-éthyle,
R₃ représente H ou un halogène,
R₂ représente H, un reste alkyle en C₁ à C₅, alkyle en C₁ à C₃ avec un noyau saturé condensé, ou aryl-(alkyle en C₁ à C₃),
R₄ est un reste aryle ou alkyle en C₁ à C₁₂,
R₅ est un reste alkyle en C₁ à C₁₂, et
R₆ est un halogène,
et leurs sels.

3. Composés de formule (I) suivant la revendication 1, dans laquelle
R₁ est un reste α-méthyle, β-méthyle, α-éthyle ou β-éthyle,
R₃ représente H, un groupe OH, le chlore, le brome, le fluor, N₃, CN, SCN ou SeCN,
R₂ dans les restes sulfamoyloxy (-OSO₂NHR₂) représente H et/ou un reste alkyle en C₁ à C₅, alcanoyle en C₁ à C₅ ou (cycloalkyle en C₃ à C₇)-carbonyle, et
R₆ représente H, le chlore, le brome ou le fluor,
et leurs sels.

4. Composés de formule (I) suivant la revendication 1, dans laquelle
R₁ est un reste β-méthyle ou β-éthyle,
R₃ représente le brome, le brome-[⁷⁶Br], le fluor, le fluor-[¹⁸F], l'iode-[¹²⁵I] ou [¹³¹I] ou l'astatine, et
R₆ représente le brome, le brome-[⁷⁶Br], le fluor ou le fluor- [¹⁸F1],
et R₂, R₄, R₅ et n ont les définitions indiquées dans la revendication 3,
et leurs sels.

5. Composé de formule (I) suivant la revendication 1, choisi dans chaque cas entre les composés :
3,17β-disulfamoyloxy-13β-éthyl-1,3,5(10)-gonatriène
3,17β-disulfamoyloxy-13β-méthyl-1,3,5(10),7(8)-gona-tétraène
3,17β-disulfamoyloxy-13β-méthyl-1,3,5(10)8,6-gona-pentaène
3,17β-disulfamoyloxy-13β-méthyl-1,3,5(10),8-gona-tétraène
3,17β-disulfamoyloxy-13β-méthyl-1,3,5(10)8,14-gona-pentaène
3,17β-disulfamoyloxy-13β-méthyl-1,3,5(10),8(14)-gona-tétraène
3,17β-disulfamoyloxy-13β-méthyl-1,3,5(10),9(11)-gona-tétraène
3,17β-disulfamoyloxy-13β-éthyl-1,3,5(10),9(11)-gonatétraène
3,17β-disulfamoyloxy-14β,15β-méthylène-13β-méthyl-1,3,5(10),8-gona-tétraène
3,17α-disulfamoyloxy-14β,15β-méthylène-13β-méthyl-1,3,5(10),8-gona-tétraène
3, 17β-disulfamoyloxy-14α,15α-méthylène-13β-méthyl-1,3,5(10),8-gona-tétraène
3,17α-disulfamoyloxy-14α,15α-méthylène-13β-méthyl-1,3,5(10),8-gona-tétraène
16α-bromo-3,17β-disulfamoyloxy-13β-méthyl-1,3,5(10)-gonatriène
16α-bromo-3,17β-disulfamoyloxy-13β-éthyl-1,3,5(10)-gonatriène
16β-bromo-3,17β-disulfamoyloxy-13β-méthyl-1,3,5 (10)-gonatriène
16α-chloro-3,17β-disulfamoyloxy-13β-méthyl-1,3,5(10)-gonatriène
16α-chloro-3,17β-disulfamoyloxy-13β-éthyl-1,3,5(10)-gonatriène
16β-chloro-3,17β-disulfamoyloxy-13β-méthyl-1,3,5 (10)-gonatriène
3,17β-disulfamoyloxy-16α-fluoro-13β-méthyl-1,3, 5 (10)-gonatriène
3,17β-disulfamoyloxy-16α-fluoro-13β-éthyl-1,3,5(10)-gonatriène
3,17α-disulfamoyloxy-13β-méthyl-1,3,5(10)-gonatriène
et leurs sels.

6. Préparation pharmaceutique, comprenant au moins un composé de formule (I) suivant la revendication 1, ainsi qu'un support pharmaceutiquement acceptable.

7. Composé de formule (I) suivant la revendication 1, destiné à des fins thérapeutiques.

8. Utilisation du composé de formule (I) suivant la revendication 1, pour la préparation d'un médicament destiné au traitement de tumeurs à dépendance hormonale.

9. Utilisation de composés de formule (I) suivant la revendication 1 pour la préparation d'une composition à usage diagnostique.
